(19) Europäisches Patentamt
European Patent Office
Office européen des brevets

(11) Publication number: **0 020 029**
**B1**

(12) # EUROPEAN PATENT SPECIFICATION

(45) Date of publication of patent specification: **02.01.85**

(21) Application number: **80301443.0**

(22) Date of filing: **01.05.80**

(51) Int. Cl.⁴: **C 07 J 71/00**, C 07 J 43/00, A 61 K 31/58, A 61 K 31/70, A 61 K 35/78

(54) **Pharmaceutical compositions comprising steroid alkaloids.**

(30) Priority: **02.05.79 AU 8621/79**

(43) Date of publication of application: **10.12.80 Bulletin 80/25**

(45) Publication of the grant of the patent: **02.01.85 Bulletin 85/01**

(84) Designated Contracting States: **BE CH DE FR GB IT LI LU NL SE**

(56) References cited:
**FR-M- 2 359**
**FR-M- 2 360**
**FR-M- 2 367**
**FR-M- 2 895**
**FR-M- 5 211**

**CHEMICAL ABSTRACTS, vol. 68, no. 5, 29-01-1968, page 1975, abstract 20709v. Columbus, Ohio, USA C. BODEA et al. "Antimitotoic activity of solasodine and some derivatives"**

**ARZNEIMITTLFORSCHUNG, vol. 26, no. 1, January 1976, Aulendorf, DE J. KULCSAR-GERGELY: "The effect of solasodine on the body temperature", pages 55-57**

**The file contains technical information submitted after the application was filed and not included in this specification**

(73) Proprietor: **ARUBA (QLD.) PTY. LTD.**
**c/o O.W. Gerke & Co. Qantas House Queen Street**
**Brisbane 4000 Queensland (AU)**

(72) Inventor: **Cham, Bill Elliott**
**772 Upper Brookfield Road**
**Upper Brookfield 4069, Queensland (AU)**
Inventor: **Gerns, Edwin Harold John**
**76 Holme Avenue**
**Boondall 4034, Queensland (AU)**
Inventor: **Gerns, Henry Harold**
**4, Buhot Street**
**Geebung 4034, Queensland (AU)**

(74) Representative: **Silverman, Warren et al**
**HASELTINE LAKE & CO. Hazlitt House 28 Southampton Buildings Chancery Lane**
**London WC2A 1AT (GB)**

(56) References cited:
**CHEMICAL ABSTRACTS, vol. 75, no. 7, 16-08-1971, page 222, abstract 47319z. Columbus, Ohio, USA K. NISHIE et al. "Pharmacology of solanine"**

**EP 0 020 029 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

CHEMICAL ABSTRACTS, vol. 59, no. 12, 9-12-1963, page 14053, abstract 14053d-h. Columbus, Ohio, USA KLAUS SCHREIBER et al. "Solanum alkaloids XXV. Identification of tomatine as a principal alkaloid of the wild potato Solanum demissum"

CHEMICAL ABSTRACTS, vol. 5, no. 20, 20-10-1911, pages 3406-3407. Columbus, Ohio, USA GUISEPPE ODDO et al. "Solanine-extracted from Solanum sodomaeum"

The Alkaloyds Vol. III, Vol. VII, Vol. X, by R.H.F. Manske & H.L. Holmes

**Description**

The present invention relates to pharmaceutical compositions comprising steroid alkaloids.

The compositions of the invention have been found to be useful in chemotherapeutic treatment of cancers, both neoplastic and granulmatous, of skin inflammations of mycotic infections such as tinea, of non-malignant dermatitis such as psoriasis, of haemorrhoids and of acne, and for other cosmetic uses.

The chemotherapy of cancers (with drugs) goes back into antiquity. Early Egyptian papyruses describe the application of various mixtures of drugs to ulcerating skin tumours. However, the emergence of chemotherapy as an effective modality of cancer treatment in modern medicine is a relatively recent development. Emphasis on chemotherapy as a primary modality for therapy has also come about with the realisation that cancer is often a systemic disease where local forms of treatment are often inadequate.

Large numbers of compounds have been studied in the search for new drugs with improved therapeutic efficacy. As a result of these efforts, several different classes of chemotherapeutic agents have been identified. The availability of a variety of drugs with different mechanisms of action and with differing host toxicities has provided a new dimension for the role of chemotherapy in the treatment of cancer.

The alkylating agents which include nitrogen mustard, cyclophosphamide, chlorambucil, molphalan, and busulfan, are amongst the oldest and most established drugs used for the treatment of cancer.

Antimetabolic agents have been defined and comprise another class of anti-cancer drugs. Methotrexate, fluoro-uracil, mercaptopurine, thioguanine, and cytosine, arabinoside are included in this class of potent anti-tumour drugs.

Yet another class of agents found to have significant anti-tumour activity are the antibiotics, products of living organisms that have profound anti-tumour properties. Among these are drugs such as actinomycin D, mitramycin, daunoribicin, mitomycin C, and adriamycin.

Folklore has provided a different type of chemotherapeutic agent, the vinca alkaloids. Vincristine and vinblastine (U.S. Patent 3,225,030 in 1965 by Eli Lilly), extracts of the periwinkle plant (Vinca Rosea), have become firmly established in the treatment of acute leukemia and other types of cancer.

The compositions of the present invention provide for another efficient chemotherapeutic agent for the treatment of cancer. The active components of compositions of the present invention are derived from extracts of the Solanum plant family. Extracts of one such plant (Solanum sodomeum) known in Australia as apple of Sodom, and incorrectly as devil's apple will be described by way of an example to illustrate the present application. This plant bears fruit which is similar in shape to, but smaller than an apple. The fruit of the plant is generally considered to be poisonous.

The present invention therefore provides a pharmaceutical compositions which comprise at least one active ingredient selected from solmargine, solasonine and mono- and diglycosides of solasodine, and pharmaceutically acceptable salts thereof, together with a pharmaceutically acceptable carrier.

The aglycone isolated from Solanum sodomeum is

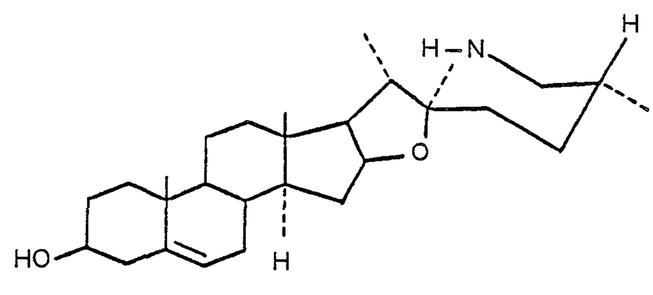

(I)

solasodine.

When the active compounds are mono- and di-glycosides of solasodine, they may be mono-, di-, tri-, tetra-, or polyglycosides and may be derived from tetroses such as erythrose and threose; pentoses such as ribose, arabinose, xylose and lyxose; and hexoses such as allose, altrose, glucose, mannose, gulose, idose, galactose, talose, fructose, sorbose, tagatose and psicose.

The sugar units in the diglycosides are linked to each other in known manner. The hexoses may, for example, have 1,5 or 1,4 linkages.

The triglycosides which can be isolated from Solanum sodomeum are solasonine (II) and solamargine (III).

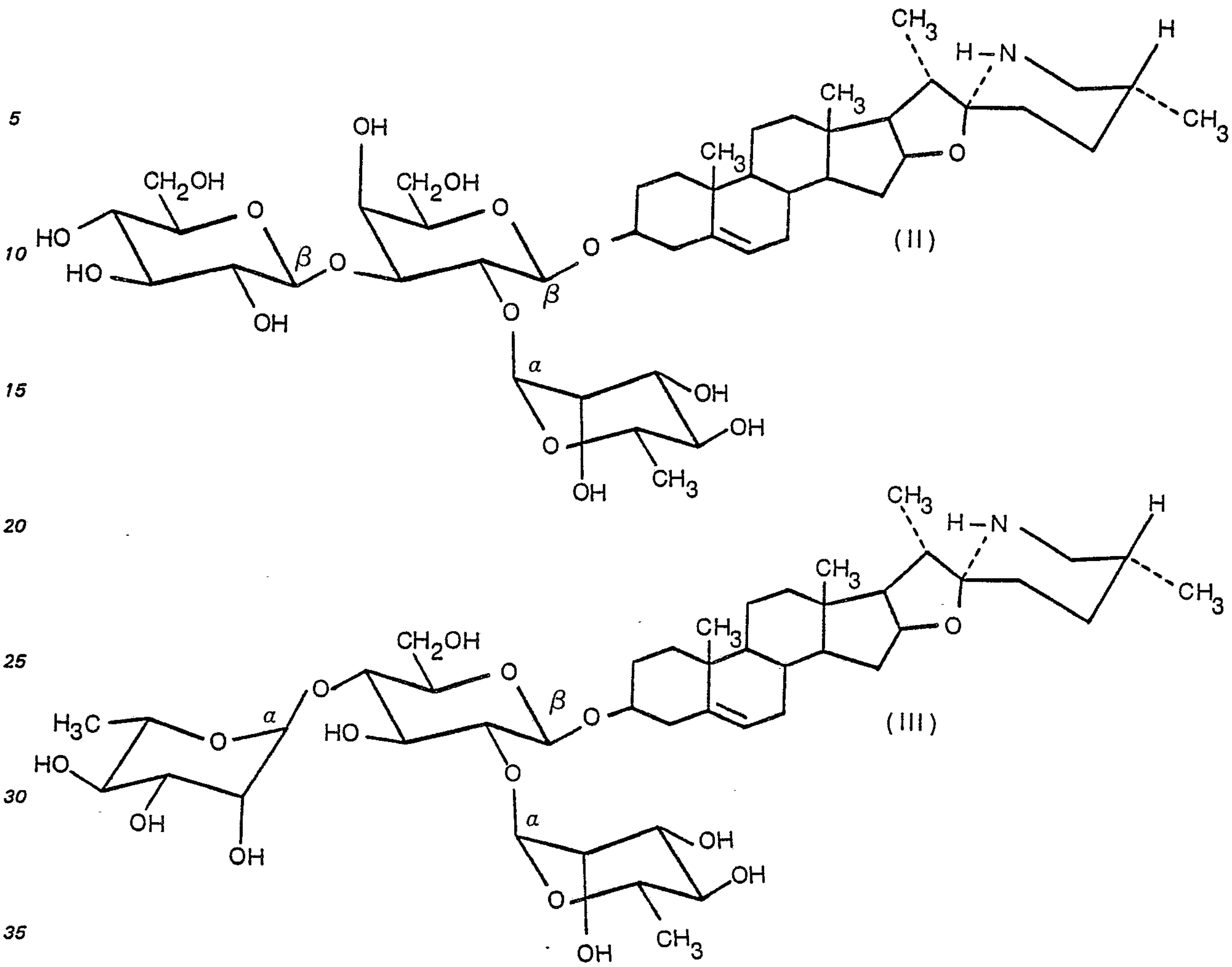

(II)

(III)

The glycoalkaloids can be extracted from plants of the Solanum species by grinding any parts of the plant and subjecting the ground plant matter to the action of dilute acid, and making the acid extracts alkaline to precipitate the glycolalkaloids.

The following examples illustrate extraction of a mixture of glycoalkaloids from Solanum sodomeum.

Example 1

Plant material from Solanum sodomeum is coarsely ground and then mixed with ten volumes (w/v) of 2% acetic (or formic) acid and shaken for two to four hours. This is then coarsely filtered (or centrifuged) and the residue is reshaken with ten volumes of 2% acetic (or formic) acid for another two to four hours at room temperature. The second extract is filtered and both solutions are added together and centrifuged to remove the last traces of residue. The solutions are made alkaline with ammonia, and heated 80°C which causes a precipitate to form. This precipitate is dissolved in boiling alcohol and filtered while boiling. The alcohol is then evaporated to dryness. This yields a fine powder.

Example 2

Plant material from Solanum sodomeum is coarsely ground with two volumes (w/v) or 3% aqueous acetic acid in a Waring blender. The mixture is diluted with another two volumes of 3% aqueous acetic acid and is then shaken for eighteen to twenty hours at room temperature, then filtered through muslin. Two litre aliquots of the filtrate is heated to 50°C with continual stirring and then concentrated ammonia solution is added until the pH = 9—10 (approx. 50 ml/litre). The solution is maintained at 50°C for a further five minutes, allowed to cool and then centrifuged. The supernatant is discarded and the precipitate is dissolved in 1 litre 3% aqueous acetic acid. The solution is centrifuged and the supernatant heated to 50°C with continual stirring. The glycoalkaloids are reprecipitated on addition of concentrated ammonia solution until the pH = 9—10. The solution is maintained at 50°C for a further five minutes as before, allowed to boil and then centrifuged. The glycoalkaloids precipitate is dried overnight at 50°C and then extracted with 100 ml boiling ethanol. The ethanolic solution is

centrifuged and the supernatant dried at 50°C. This yields a fine powder.

The extract as prepared in Examples 1 and 2 will hereinafter be referred to as BEC 001.

The following example illustrates separation of the glycoalkaloids (II) and (III) and the mono- and diglycosides of solasodine (I) from BEC 001.

Example 3

The powder extract from Solanum sodomeum (BEC 001) was dissolved in an eluant containing acetonitrile/$B_5$ (Pic Reagent)/triethanolamine (83:17:0:1) adjusted to pH 2.7—3.0 with concentrated phosphoric acid, at a concentration of 0.1% (by weight).

50 $\mu$l samples were applied to an injector (Model U6K universal injector*) and was chromatographed at ambient temperature on a 30 cm × 3.9 mm "carbohydrate analysis" or an "—$NH_2$" prepacked column*, average particle size 10 $\mu$m at a flow rate of 2 ml per minute (Model 6000A solvent delivery system*). A model 450 variable wavelength detector*, with sensitivity set at 0.01 Aufs, was used and peak areas at 205 nm were recorded with a 10 mV omniscribe recorder (*Equipment obtained from Waters Associates Inc.).

Suitable pharmaceutically acceptable salts of the active compounds thus separated are, e.g. the hydrochlorides and hydrogen sulfates.

The compositions of the present invention may be used in the chemotherapeutic treatment of cancers, both neoplastic and granulomatous, of skin inflammation, of mycotic infections such as tinea and ringworms, of viral infections such as warts, of haemarrhoids, of bacterial infections such as acne, and of non malignant dermatitis such as psoriasis, and for other cosmetic uses. They may also be used as adjuncts to topical antifungal and antibacterial preparations.

The carrier material may be an organic or inorganic inert carrier material suitable for internal (e.g. oral), external (e.g. dermal), or parenteral administration. Examples of such carrier materials include dimethylsulfoxide, water, lactose, starch, magnesium stearate, talc, gum arabic, gelatin, polyalkylene glycols and petroleum jelly. The pharmaceutical preparations can be made up in a solid form, e.g., tablets, dragees, suppositories or capsules, or in liquid form, e.g., as solutions, emulsions, suspensions or aerosols. The pharmaceutical preparations may be subjected to customary pharmaceutical operations, such as sterilisation and may contain adjuvants such as preservatives, stabilisers, wetting agents, buffers and salts for varying osmotic pressure.

More particularly the steroid alkaloids may be formulated in suitable pharmaceutical vehicles for topical application e.g., as lotions, ointments or creams by incorporating them in conventional lotion, ointment or cream bases, such as zinc oxide, alkyl polyether alcohols, cetyl alcohol, stearyl alcohol or polyethylene glycol 1000 monoacetyl ether (cetomacrogol). They may also be formulated as solutions in dimethylsulfoxide. Other solid forms include powders wherein the steroid alkaloids are incorporated in conventional powder bases such as starch or talc or gels in which the base is, e.g. glycerol or tragacanth.

The following example illustrates toxicity tests on BEC 001 as prepared in Examples 1 and 2.

Example 4

Single Dose

The toxicity of a single ip dose of BEC 001 in mice is illustrated in Figure 1. It can be seen that the $LD_{50} = 30$ mg/kg (mg BEC 001 per kg mouse body weight). Toxicity studies of glycoalkaloids extracted from Solanum turburosum which contains mainly solanine were conducted by Patil et al., (Food and Cosmetics Toxicology 10,395 (1972)) who found an $LD_{50}$ in mice (ip administration) of 32.3 mg/kg. Nishie et al (Toxicology and Applied Pharmacology 19,81 (1971)) reported an ip $LD_{50}$ of 42±1.8 mg/kg in mice, whereas Gull et al (Hortscience, 5,316 (1970)) found an ip $LD_{50}$ of 75 mg/kg when the pure alkaloid was used. The values calculated from experimental data on BEC 001 agree closely with those of Patil et al and Nishie et al. The postmortem examinations following administration of BEC 001, like those reported by Gull et al, revealed no well-defined symptoms directly attributable to the toxic effect of the glycoalkaloids. Patil et al found that administration of an ip dose in mice of over 50 mg solanine/kg was lethal within 1—3 hr, but a dose of 10 mg/kg caused no deaths; this is in agreement with results found. It was found that the $LD_{50}$ for gastric intubation in mice was 550 mg BEC 001 per kilogram, this is in close agreement with the value reported by Gull et al of 590 mg solanine per kilogram.

The $LD_{50}$ of BEC 001 for rats was found to be 41 mg/kg.

Multiple Doses

The $LD_{60}$ for mice by 14 ip administrations over 14 days (one injection per day) was 10 mg/kg.

The $LD_{50}$ for rats by 8 ip administrations over 8 days (one injection per day) was 20 mg/kg.

Local toxicity in mice

Intact and abraded skin.

The hair on the back of mice was clipped with electric clippers. The area of the hair removed was about 2.5 cm × 5.0 cm. Care was taken to avoid injury in a batch of 24 mice (batch A). Superficial skin

injury using electric clippers was deliberately done on another batch of 24 mice (batch B). The average weight of mice was initially 40 g per mouse (36—44 g). Batch A was divided into $A_1$ and $A_2$ of 12 mice each. Similarly batch B was divided into $B_1$ and $B_2$. Ten microlitres of dimethylsulfoxide (DMSO) was applied daily to the backs of mice in batches $A_1$ and $B_1$. Ten microlitres of DMSO containing 5% BEC 001 was applied to batches $A_2$ and $B_2$ daily. This study was continued for 16 weeks. At the end of the 16 weeks all mice grew normal hair and the skin appeared normal. A group of 5 mice of each batch was then sacrificed. Post mortem investigation revealed that there was no apparent gross abnormality of thoracic or abdominal contents.

Daily doses of approximately 12.5 mg BEC 001 per kg body weight applied topically to intact or abraded skin of mice for 16 weeks did not produce any obvious adverse effects.

Side effects

Patil et al reported that solanine appeared to be a weak-to-moderate inhibitor of both specific and non-specific cholinesterase. Following small multiple doses of solanine, a quick inhibition followed by rapid recovery of serum cholinesterase was noted in the dog. Red-cell cholinesterase was not inhibited. It was speculated that while small does of solanine may cause discomfort upon ingestion, repeated doses will have little noticeable effect resulting from acetylcholinesterase inhibition. Further studies by Patil et al revealed that atropine appeared to be antagonistic to the toxicity of solanine.

In human experiments Ruhl (Archiv der Pharmazie 284,67 (1961)) indicated that an oral dose of 200 mg solanine caused hyperesthesia, drowsiness, itchiness in the neck region, and dyspnea: higher doses caused vomiting and diarrhoea. It has been found that an oral dose of 250 mg BEC 001 caused no such effects in a human subject.

The following example illustrates the effect of BEC 001 on sarcoma 180 activity in mice.

Example 5

A suspension of sarcoma 180 cells was taken from a peritoneal fluid of a host mouse and a known aliquot was injected ip into the experimental mice to produce sarcoma 180 activity in the ascitic fluid. BEC 001 was made up as a 5% stock solution in dimethylsulfoxide (DMSO). The necessary control experiments was also conducted. Treatment of the mice was done by ip injections with appropriate quantities of BEC 001.

Figure 2 illustrates the inhibition of the activity of sarcoma 180 by BEC 001 (expressed as % survival of mice due to BEC 001). Mice inoculated with sarcoma 180 cells generally died in two to three weeks after inoculation. The criterion of survival was arbitrarily taken as eight weeks. This is 4 times normal time to death after inoculation of sarcoma 180 control mice untreated with BEC 001. It can be seen in Figure 2 that a single administration of BEC 001 has an $ED_{50}$ of 10 mg/kg (i.e. activity of sarcoma 180 was inhibited in 50% of the animals of this dose).

Inhibition of the lethal effect of sarcoma 180 activity in mice was dependent on the number of doses of BEC 001. Figure 3 depicts the effect of the number of administrations of BEC 001 at a concentration of 8 mg/kg on the inhibition of the mice from this cancer type. Two doses achieved 42% inhibition whereas with three and four doses almost complete inhibition was obtained.

Figure 4 illustrates the results obtained in the test on the inhibition of sarcoma 180 activity in the ascitic fluid of recipient mice using BEC 001. Five groups of twelve mice were used in the test. Curve △ represents 1 injection of 8 mg/kg; Curve ● represents 2 injections of 8 mg/kg; Curve □ represents 3 injection of 8 mg/kg; Curve ▲ represents 4 injections of 8 mg/kg. The ip injections were done on consecutive days.

In summary; these preliminary studies indicate that BEC 001 is very effective in producing a highly significant inhibitory activity on the terminal cancer sarcoma 180. The efficacy and toxicity of BEC 001 depends on the route and type of application and is species dependent.

The following example illustrates the effect of BEC 001 on skin rumours.

Example 6

Preliminary studies of BEC 001 have shown exceedingly promising results in the treatment of skin cancers. Various preparations have been investigated ranging from crude plant extract (macerated fruit), BEC 001 in DMSO, BEC 001 in paraffin, BEC 001 in zinc ointment, BEC 001 in zinc cream, and BEC 001 in cetomacrogol. Studies done so far on a limited number of patients with skin rumours indicate that BEC 001 is effective to the types studied, viz, keratoses, basal cell carcinoma (BCC), and squamous cell carcinoma (SCC).

Concentrations ranging from 0.1% to 50% BEC 001 have been studied on the skin in man. No apparent side effects were observed.

The preliminary studies indicate that the following formulations may be used to obtain satisfactory results.

| | |
|---|---|
| BEC 001 | 40% |
| DMSO | 5% |
| Cetomacrogol | 91% all in W/W |

One subject who originally had over 200 skin tumours has been using this preparation for over ten months and as far as can be determined has shown no ill effects.

One investigator has been applying 80 mg BEC 001, in the ointment form, twice daily for three months on himself and has encountered no apparent ill effects. An oral dose of 200 mg of BEC 001 did not affect the subject adversely. Biochemical (SNAC 20) and haematological (coulter screen, platelet count, differential W.C.C.) tests revealed no significant changes due to BEC 001.

Two grams of the ointment are adequate to apply to 25 cm $\times$ 25 cm = 625 $cm^2$ area on the skin in man. This corresponds to 1.6 mg glycoalkaloid per kg body weight for a person weighing 50 kg. This refers to topical application. The absorption kinetics of this drug through the skin is not known at present.

Oral toxicity studies in mice have shown that the $LD_{50}$ is approximately 550 mg/kg.

Daily doses of approximately 12.5 mg BEC 001 per kg body weight applied topically to intact or abraded skin of mice for 16 weeks did not produce any obvious adverse effects.

## Claims

1. A pharmaceutical composition in dosage unit form which comprises at least one active ingredient selected from solamargine, solasonine and mono- and diglycosides of solasodine, and pharmaceutically acceptable salts thereof, together with a pharmacologically acceptable carrier.

2. A pharmaceutical composition intended for the treatment of cancer, skin inflammation, tinea, warts, acne, psoriasis, haemorroids or other skin infection, which comprises at least one active ingredient selected from solamargine, solasonine and mono- and diglycosides of solasodine, and pharmaceutically acceptable salts thereof.

3. A pharmaceutical composition for topical application, which comprises at least one active ingredient selected from solamargine, solasonine and mono- and diglycosides of solasodine and pharmaceutically acceptable salts thereof in association with a pharmacologically acceptable carrier appropriate to topical application of said ingredient.

4. A composition as claimed in claim 3, which is in the form of a lotion, ointment, cream, solution in dimethylsulphoxide, powder or gel.

5. A composition as claimed in any one of the preceding claims, which comprises as its active ingredient a mixture of solamargine, solasonine and mono- and diglycosides of solasodine.

6. A composition as claimed in claim 5, wherein said mixture is extracted from the plant Solanum sodomeum by grinding parts of said plant, subjecting the ground plant matter to the action of formic acid and making the acid extract alkaline to precipitate said mixture.

7. A composition as claimed in claim 5, wherein said mixture is extracted from the plant Solanum sodomeum by the steps of:—

(i) grinding said plant material coarsely;
(ii) mixing said ground plant material with dilute acid to form a first acid extract;
(iii) separating the solid residue from step (ii) from said first acid extract;
(iv) mixing said solid residue with further dilute acid to form a second acid extract;
(v) separating the solid residue from step (iv) from said second acid extract;
(vi) combining said first and second acid extracts; and
(vii) adding alkali to said combined acid extracts to precipitate said glycoalkaloids.

8. A composition as claimed in claim 7 wherein said dilute acid is 2% acetic acid or 2% formic acid.

9. A composition as claimed in claim 7 or 8, wherein either or both of step (ii) and step (iv) is carried out at ambient temperature for between 2 and 4 hours.

10. A composition as claimed in any one of claims 7 to 9, wherein said alkali is ammonia.

11. A composition as claimed in any one of claims 7 to 10, in which said mixture has been extracted as specified in a said claim with the additional step of heating the combined extracts which have been made alkaline in step (vii) to 80°C.

12. A composition as claimed in any one of claims 7 to 11 wherein said glycoalkaloids so obtained have been purified by recrystallisation.

13. A composition as claimed in claim 5, wherein said mixture is extracted from the plant Solanum sodomeum by the steps of:—

(a) grinding said plant material in the presence of a dilute acid;
(b) diluting the mixture formed thereby with further dilute acid;
(c) agitating said diluted mixture to form an acid extract;
(d) separating the solid residue from step (c) from said acid extract;

(e) warming the said acid extract; and
(f) increasing the pH of said warmed acid extract to 9 to 10 to precipitate said glycolalkaloids.

14. A composition as claimed in claim 13, wherein, in extracting said mixture, there have been carried out, in addition to said steps, the further steps of:—
(g) dissolving said glycolalkaloids in dilute acid;
(h) separating any material which does not dissolve;
(i) warming the resultant solution;
(j) increasing the pH of said warmed solution to 9 to 10 to precipitate said glycoalkaloids;
(k) holding the suspension formed thereby at the temperature to which it is warmed for a short period;
(l) boiling said suspension; and
(m) separating the precipitated glycolalkaloids.

15. A composition as claimed in claim 14, wherein the glycoalkaloids so obtained are purified by recrystallisation.

16. A composition as claimed in any one of claims 13 to 15 wherein said dilute acid in any one or more of steps (a), (b), and (g) is 3% acetic acid.

17. A composition as claimed in any one of claims 13 to 16, wherein step (c) is carried out at ambient temperature for between 18 and 24 hours.

18. A composition as claimed in any one of claims 13 to 17, wherein in one or more of steps (e), (i) and (k), warming to 50°C is effected.

19. A composition as claimed in any one of claims 13 to 18 wherein in step (f) and/or (j), the pH is increased by addition of ammonia.

20. A composition as claimed in any one of claims 14 to 19, wherein said short period is 5 minutes.

**Patentansprüche**

1. Eine pharmazeutische Zusammensetzung in dosierbarer einheitlicher Form (in dosage unit form) enthaltend wenigstens einen aktiven Bestandteil ausgewählt aus der Gruppe Solamargin, Solasonin sowie Mono- und Diglykoside von Solasodin und deren pharmazeutisch annehmbaren Salzen und einen pharmakologisch annehmbaren Träger.

2. Eine pharmazeutische Zusammensetzung bestimmt für die Behandlung von Krebs, Hautentzündungen, Tinea, Warzen, Akne, Psoriasis, Hämmorhoiden und anderen Hautinfektionen, welches enthält wenigstens einen aktiven Bestandteil ausgewählt aus der Gruppe Solamargin, Solasonin und Mono- bzw. Diglykosiden von Solasodin sowie deren pharmazeutisch annehmbaren Salzen.

3. Eine pharmazeutische Zusammensetzung für lokale Anwendung, welche enthält wenigstens einen aktiven Bestandteil ausgewählt aus der Gruppe Solamargin, Solasonin und Mono- bzw. Diglykosiden von Solasodin sowie deren pharmazeutisch annehmbaren Salzen in Verbindung mit einem pharmakologisch annehmbaren Träger, der geeignet ist für die lokale Anwendung des besagten aktiven Bestandteils.

4. Eine Zusammensetzung nach Patentanspruch 3, vorliegend in Form einer Lotion, einer Salbe, einer Creme, einer Lösung in Dimethylsulfoxid, eines Pulvers oder eines Gels.

5. Eine Zusammensetzung nach jedem der vorherigen Patentansprüche, enthaltend als aktiven Bestandteil eine Mischung von Solamargin, Solasonin und Mono- bzw. Diglykosiden von Solasodin.

6. Eine Zusammensetzung nach Patentanspruch 5, wobei die Mischung aus der Pflanze Solanum sodomeum extrahiert worden ist durch Vermahlen von Teilen dieser Pflanze, Behandeln des Mahlgutes mit Ameisensäure und Alkalischstellung des sauren Extrakts zum Ausfällen der besagten Mischung.

7. Eine Zusammensetzung nach Patentanspruch 5, wobei die Mischung aus der Pflanze Solanum sodomeum durch folgende Schritte extrahiert worden ist:
(i) grobes Vermahlen des besagten Pflanzenmaterials,
(ii) Vermischen des vermahlenen Pflanzenmaterials mit verdünnter Säure unter Bildung eines ersten sauren Extrakts,
(iii) Abtrennen des festen Rückstandes aus der Stufe (ii) von besagtem ersten sauren Extrakt,
(iv) Vermischen des besagten festen Rückstandes mit weiterer verdünnter Säure unter Bildung eines zweiten sauren Extrakts,
(v) Abtrennen des festen Rückstandes aus der Stufe (iv) von besagtem zweiten sauren Extrakt,
(vi) Vereinigen der besagten ersten und zweiten sauren Extrakte und
(vii) Zugabe von Alkali zu den besagten vereinigten sauren Extrakten zur Ausfällung der besagten Glykoalkaloide.

8. Eine Zusammensetzung nach Patentanspruch 7, wobei die besagte verdünnte Säure eine 2%ige Essigsäure oder 2%ige Ameisensäure ist.

9. Eine Zusammensetzung nach Patentanspruch 7 oder 8, wobei entweder eine oder beide Stufen (ii) und (iv) bei Raumtemperatur während 2 bis 4 Stunden durchführt worden sind.

10. Eine Zusammensetzung nach einem der Patentansprüche 7 bis 9, wobei das besagte Alkali Ammoniak ist.

11. Eine Zusammensetzung nach einem der Patentansprüche 7 bis 10, wobei die besagte Mischung, welche in der beschriebenen Weise extrahiert worden ist, zusätzlich noch in der Weise behandelt worden ist, daß die vereinigten Extrakte nach der Alkalischstellung in Stufe (vii) bis 80°C erwärmt werden.

12. Eine Zusammensetzung nach einem der Patentansprüche 7 bis 11, wobei die besagten Glykoalkaloide noch durch Umkristallisieren gereinigt worden sind.

13. Eine Zusammensetzung nach Patentanspruch 5, wobei die besagte Mischung aus der Pflanze Solanum sodomeum durch folgende Schritte extrahiert worden ist:
(a) Vermahlen des besagten Pflanzenmaterials in der Gegenwart einer verdünnten Säure,
(b) Verdünnen der gebildeten Mischung mit weiterer verdünnten Säure,
(c) Rühren der besagten verdünnten Mischung zur Bildung eines sauren Extrakts,
(d) Abtrennen des festen Rückstandes aus der stufe (c) von besagtem sauren Extrakt,
(e) Erwärmen des besagten sauren Extrakts und
(f) Erhöhen des pH- Wertes vom erwärmten sauren Extrakt auf 9 bis 10 zum Ausfällen der besagten Glykoalkaloide.

14. Eine Zusammensetzung nach Patentanspruch 13, wobei zusätzlich zu den dort beschriebenen Stufen die folgenden weiteren Stufen durchgeführt worden sind:
(g) Auflösen der besagten Glykoalkaloide in verdünnter Säure,
(h) Abtrennen jeglichen Materials, das sich nicht löst,
(i) Erwärmen der sich ergebenden Lösung,
(j) Erhöhen des pH-Wertes der besagten erwärmten Lösung auf 9 bis 10 zur Ausfallung der besagten Glykoalkaloide,
(k) Halten der so gebildeten Suspension bei der Temperatur für eine kurze Zeit,
(l) Sieden (boiling) der Suspension und
(m) Abtrennen der ausgefällten Glykoalkaloide.

15. Eine Zusammensetzung nach Patentanspruch 14, wobei die erhaltenen Glykoalkaloide nach durch Umkristallisieren gereinigt sind.

16. Eine Zusammensetzung nach einem der Patentansprüche 13 bis 15, wobei die verdünnte Säure in einer oder mehreren der Stufen (a), (b) und (g) 3%ige Essigsäure ist.

17. Eine Zusammensetzung nach einem der Patentansprüche 13 bis 16, wobei die Stufe (c) bei Raumtemperatur während 18 bis 24 Stunden durchgeführt worden ist.

18. Eine Zusammensetzung nach einem der Patentansprüche 13 bis 17, wobei in einer oder mehreren Stufen (e), (i) und (k) ein Erwärmen bis 50°C durchgeführt worden ist.

19. Eine Zusammensetzung nach einem der Patentansprüche 13 bis 18, wobei in der Stufe (f) und/oder (j) der pH- Wert durch Zusatz von Ammoniak erhöht worden ist.

20. Eine Zusammensetzung nach einem der Patentansprüche 14 bis 19, wobei die besagte kurze Zeit 5 Minuten ist.

**Revendications**

1. Composition pharmaceutique sous forme d'unités posologiques qui comprend au moins un ingrédient actif choisi parmi la solamargine, la solasonine et des mono- et diglycosides de solasodine, et leurs sels pharmaceutiquement acceptables, ainsi qu'un véhicule acceptable en parmacologie.

2. Composition pharmaceutique envisagée pour le traitement du cancer, les inflammations de la peau, la teigne, les verrues, l'acné, le psoriasis, les hémorroïdes ou autres infections de la peau, qui comprend au moins un ingrédient actif choisi parmi la solomargine, la solasonine et les mono- et diglycosides de solasodine, et leurs sels pharmaceutiquement acceptables.

3. Composition pharmaceutiquement pour application topique qui comprend au moins un ingrédient actif choisi parmi la solamargine, la solasonine et les mono- et diglycosides de solasodine et leurs sels pharmaceutiquement acceptables en association avec un véhicule acceptable en pharmacologie approprié pour l'application topique dudit ingrédient.

4. Composition telle que revendiquée dans la revendication 3, qui est sous la forme d'une lotion, de pommade, de crème, de solution dans le diméthylsulfoxyde, de poudre ou de gel.

5. Composition telle que revendiquée dans l'une quelconque des revendications précédents qui comprend comme ingrédient actif un mélange de solamargine, de solasonine et de mono- et diglycosides de solasodine.

6. Composition telle que revendiquée dans la revendication 5, dans laquelle ledit mélange est extrait de la plante Solanum sodomeum en broyant des parties de ladite plante, en soumettant la matière broyée de la plante à l'action de l'acide formique et en rendant alcalin l'extrait à l'acide pour précipiter ledit mélange.

7. Composition telle que revendiquée dans la revendication 5, dans laquelle ledit mélange est extrait de la plante Solanum sodomeum par les opérations suivantes:
(i) broyage grossier de ladite matière de la plante;
(ii) mélangeage de ladite matière broyée de la plante avec un acide dilué pour former un premier extrait acide;
(iii) séparation du résidu solide provenant du stade (ii) à partir dudit premier extrait acide;

(iv) mélangeage dudit résidu solide avec encore de l'acide dilué pour former un second extrait acide;
(v) séparation du résidu solide du stade (iv) à partir dudit second extrait acide;
(vi) combinaison desdits premier et second extraits acides et
(vii) addition d'un produit alcalin auxdits extraits acides combinés pour précipiter lesdits glycoalcaloïdes.

8. Composition telle que revendiquée dans la revendication 7, dans laquelle ledit acide dilué est l'acide acétique à 2% ou l'acide formique à 2%.

9. Composition telle que revendiquée dans la revendication 7 ou 8, dans laquelle l'un des stades (ii) et (iv), ou les deux, est effectué à la température ambiante entre 2 et 4 heures.

10. Composition telle que revendiquée dans l'une quelconque des revendications 7 à 9, dans laquelle ledit produit alcalin est l'ammoniaque.

11. Composition telle que revendiquée dans l'une quelconque des revendications 7 à 10, dans laquelle ledit mélange a été extrait comme mentionné dans une revendication précédente en effectuant en plus un stade de chauffage à 80°C des extraits combinés qui ont été rendus alcalins dans le stade (vii).

12. Composition telle que revendiquée dans l'une quelconque des revendications 7 à 11, dans laquelle lesdits glycoalcaloïdes ainsi obtenus ont été purifiés par recristallisation.

13. Composition telle que revendiquée dans la revendication 5, dans laquelle ledit mélange est extrait de la plante Solanum sodomeum par les stades suivants:
(a) broyage de ladite matière de la plante en présence d'un acide dilué;
(b) dilution du mélange ainsi formé avec une quantité supplémentaire d'acide dilué;
(c) agitation dudit mélange dilué pour former un extrait acide;
(d) séparation du résidu solide provenant du stade (c) à partir dudit extrait acide;
(e) chauffage dudit extrait acide; et
(f) augmentation du pH à 9—10 dudit extrait acide chauffé pour précipiter lesdits glycoalcaloïdes.

14. Composition telle que revendiquée dans la revendication 13, dans laquelle dans l'extraction dudit mélange ont été effectués, en plus desdits stades les stades suivants:
(g) dissolution desdits glycoalcaloïdes dans l'acide dilué;
(h) séparation de toute matière insoluble;
(i) chauffage de la solution résultante;
(j) augmentation du pH à 9—10 de ladite solution chauffée pour précipiter lesdits glycoalcaloïdes;
(k) maintient de la suspension ainsi formée à la température à laquelle elle a été chauffée pendant une courte période;
(l) ébullition de ladite suspension; et
(m) séparation des glycoalcaloïdes précipités.

15. Composition telle que revendiquée dans la revendication 14, dans laquelle les glycoalcaloïdes ainsi obtenus sont purifiés par recristallisation.

16. Composition telle que revendiquée dans l'une quelconque des revendications 13 à 15, dans laquelle ledit acide dilué dans l'un quelconque ou dans plusieurs des stades (a), (h) et (g) est l'acide acétique à 3%.

17. Composition telle que revendiquée dans l'une quelconque des revendications 13 à 16 dans laquelle le stade (c) est effectué à la température ambiante entre 18 et 24 heures.

18. Composition telle que revendiquée dans l'une quelconque des revendications 13 à 17, dans laquelle le chauffage est effectué à 50°C dans un ou plusieurs des stades (e), (i) et (k).

19. Composition telle que revendiquée dans l'une quelconque des revendications 13 à 18, dans laquelle le pH est augmenté en ajoutant de l'ammoniaque dans les stades (f) et/ou (j).

20. Composition telle que revendiquée dans l'une quelconque des revendication 14 à 19, dans laquelle ladite période courte est de 5 minutes.

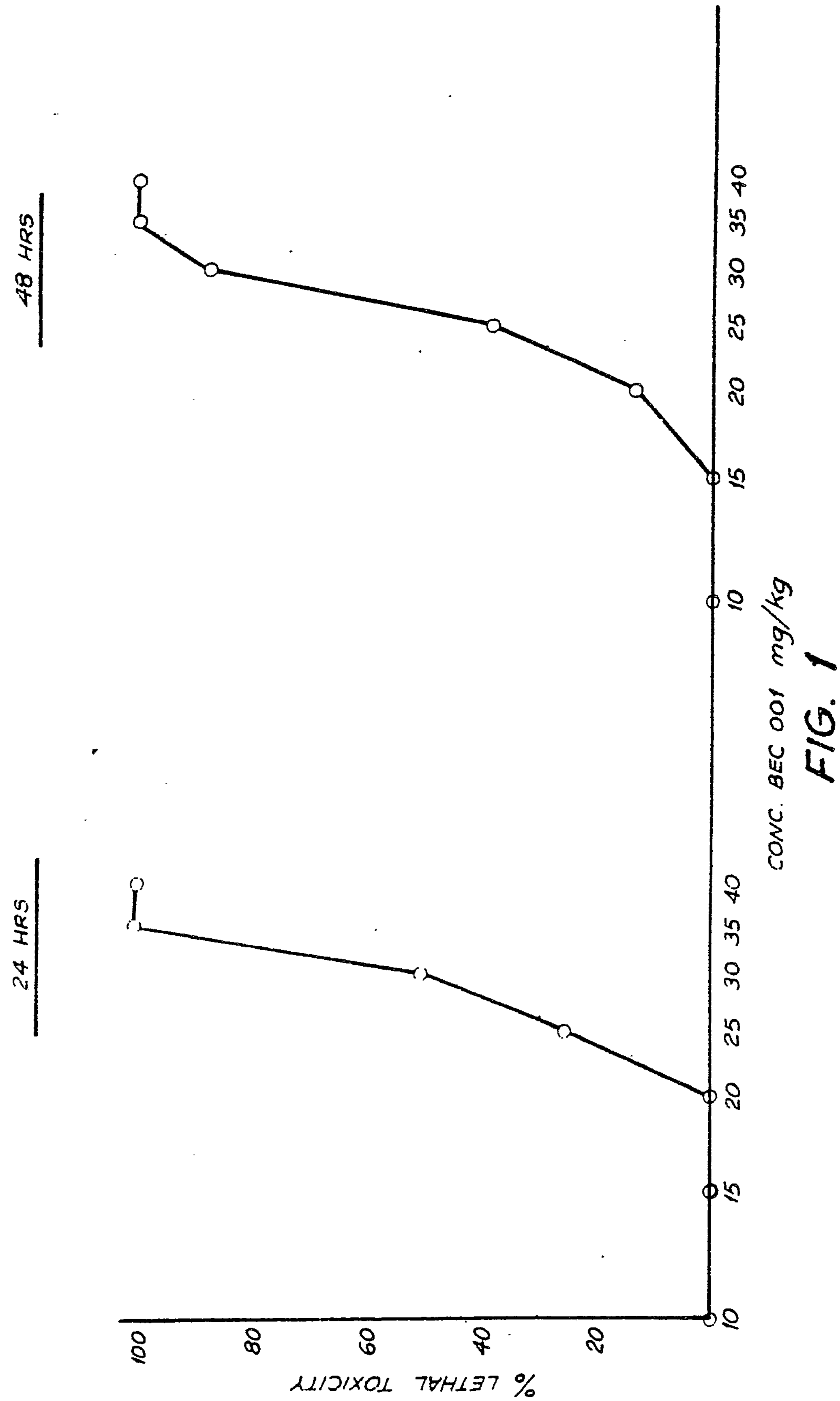
24 HRS
48 HRS
10
15
20
25
30
35
40
CONC. BEC 001 mg/kg
100
80
60
40
20
% LETHAL TOXICITY

FIG. 1

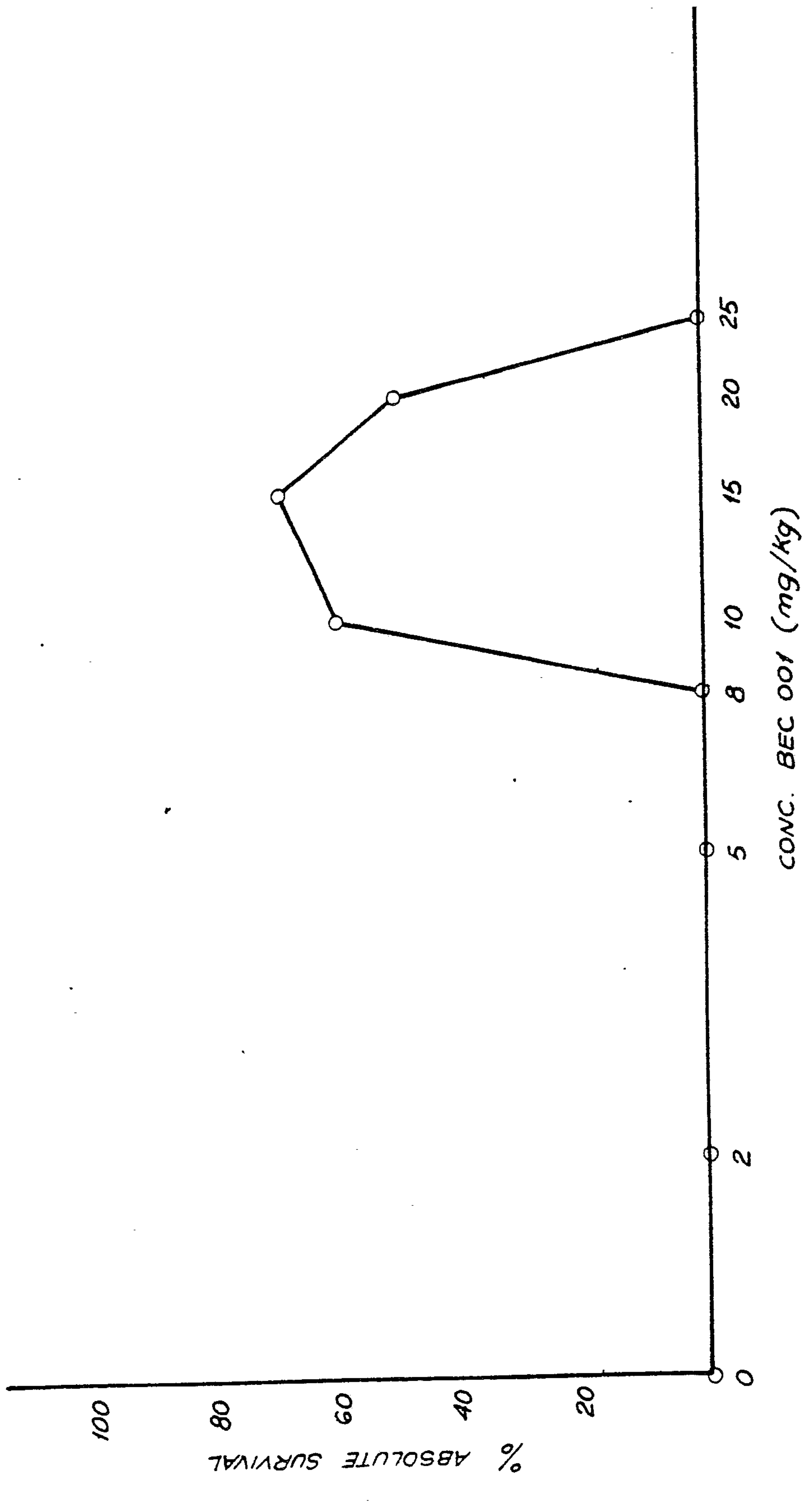

0
2
5
8
10
15
20
25
CONC. BEC 001 (mg/kg)
20
40
60
80
100
% ABSOLUTE SURVIVAL


FIG. 2

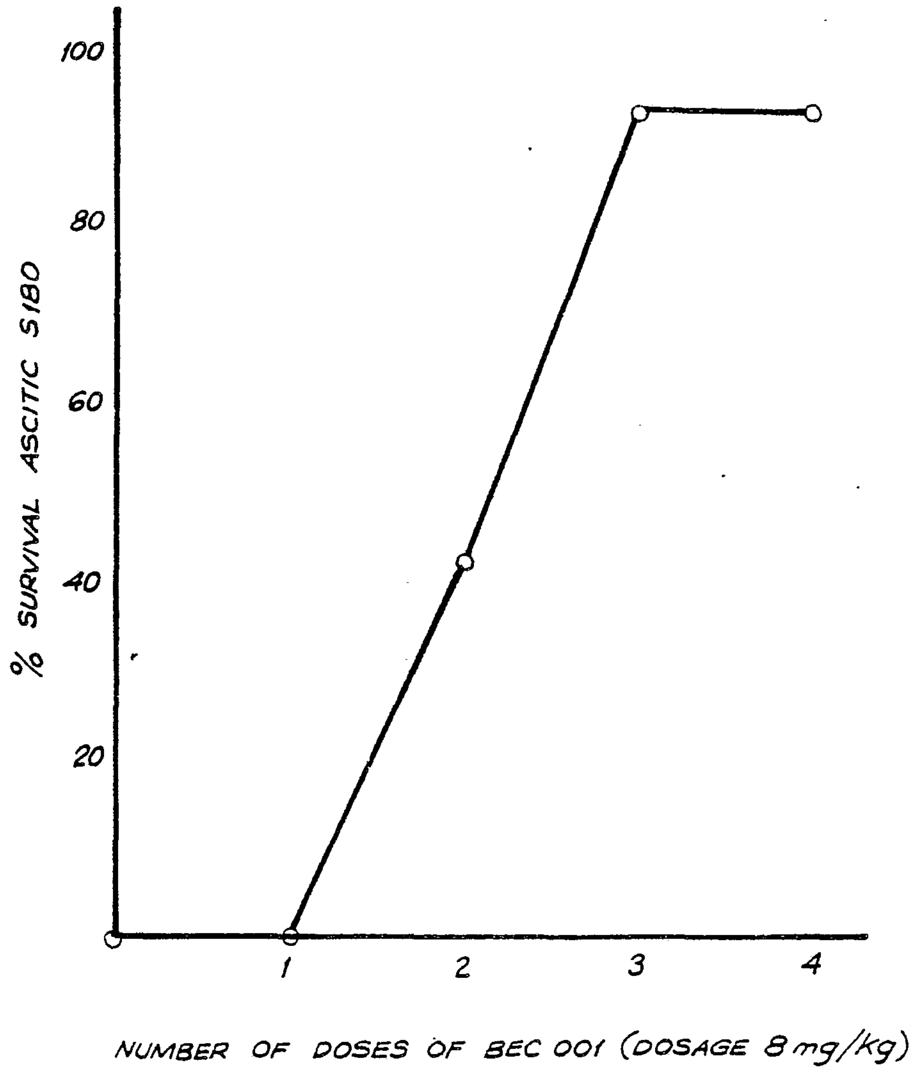
100
80
60
40
20
% SURVIVAL ASCITIC S180
1
2
3
4
NUMBER OF DOSES OF BEC 001 (DOSAGE 8 mg/kg)

FIG. 3

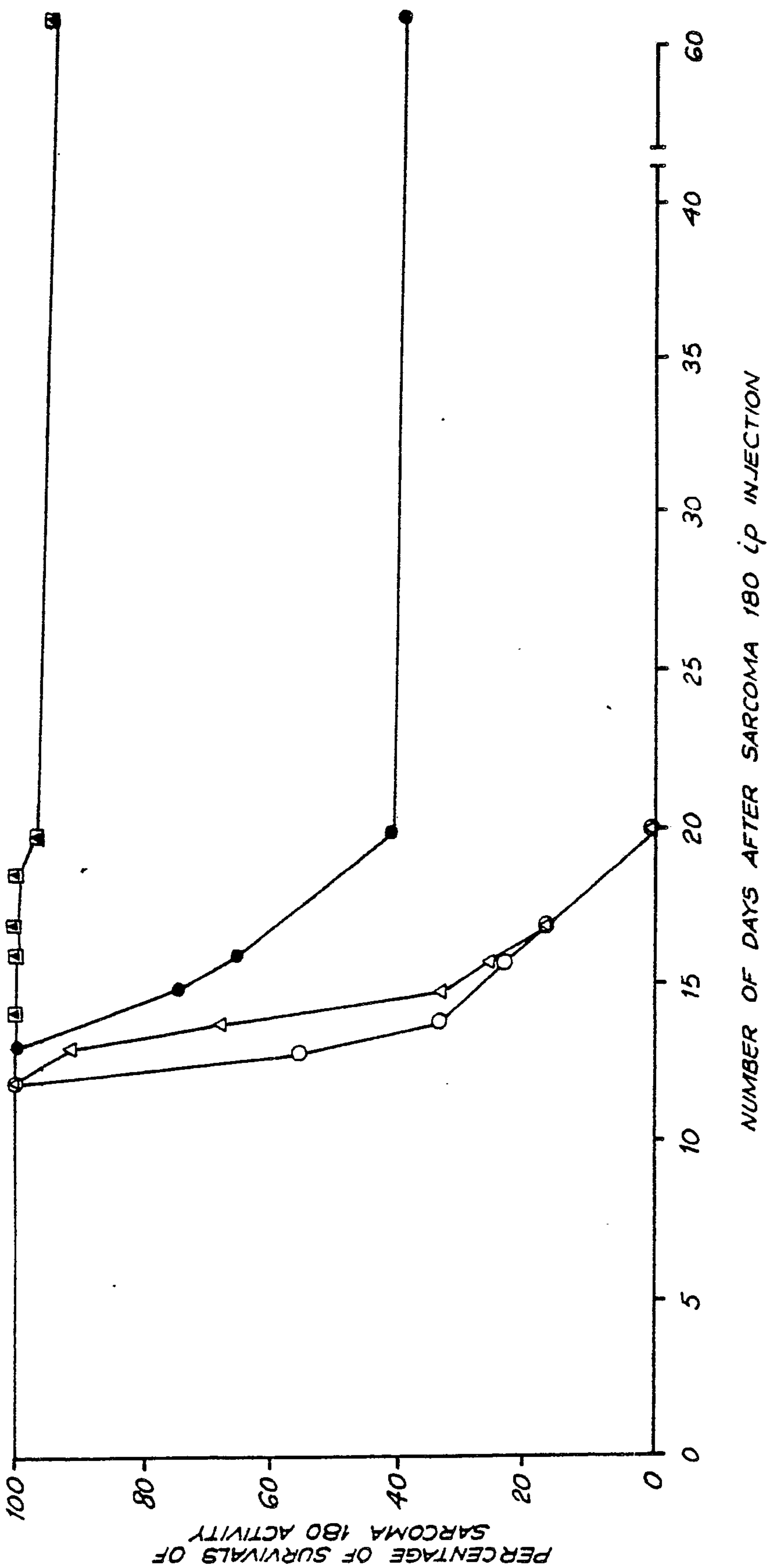
PERCENTAGE OF SURVIVALS OF
SARCOMA 180 ACTIVITY
100
80
60
40
20
0
0
5
10
15
20
25
30
35
40
60
NUMBER OF DAYS AFTER SARCOMA 180 ip INJECTION

FIG. 4